# EUROPÄISCHE PATENTSCHRIFT

(11) **EP 1 392 813 B1**
(45) Veröffentlichungstag und Bekanntmachung des Hinweises auf die Patenterteilung: **10.05.2006**
(21) Anmeldenummer: 02740687.5
(22) Anmeldetag: 05.06.2002
(51) Int. Cl.: C12M 1/00

(54) **BIOREAKTOR MIT WENIGSTENS ZWEI REAKTIONSKAMMERN**
BIOREACTOR HAVING AT LEAST TWO REACTION CHAMBERS
BIOREACTEUR COMPORTANT AU MOINS DEUX CHAMBRES DE REACTION

(30) Priorität: 08.06.2001 DE 10127869
(43) Veröffentlichungstag der Anmeldung: 03.03.2004
(73) Patentinhaber: Höfer Bioreact GmbH, 53115 Bonn (DE)
(72) Erfinder: HÖLKER, Udo, 53639 Königswinter-Rauschendorf (DE)
(74) Vertreter: von Kirschbaum, Alexander
(86) Internationale Anmeldenummer: PCT/EP2002/006159
(87) Internationale Veröffentlichungsnummer: WO 2002/100999

(56) Entgegenhaltungen:
- WO-A-01/19954
- WO-A-86/05202
- WO-A-92/01779

## Beschreibung

Die Erfindung betrifft einen Bioreaktor mit wenigstens zwei Reaktionskammern zur Fermentation flüssiger und/oder fester Stoffe, ein Fermentationsverfahren mit wenigstens zwei Reaktionsansätzen unter Verwendung des genannten Bioreaktors, welches insbesondere zum Optimieren und/oder Upscalen von Fermentationsreaktionen geeignet ist.

Ein Bioreaktor zur Fermentation von festen Stoffen ist in WO 01/19954 beschrieben. Dieser Bioreaktor weist einen Fermentationsbehälter auf, in den über eine Zugabeeinrichtung bioreaktive Stoffe zugeführt werden können. Ferner ist eine aus mehreren Leitungsrohren bestehende Düsenanordnung vorgesehen, die in den Fermentationsbehälter hineinragt und in diesen ein- und ausfahrbar ist. Durch die Düsenanordnung können an dem Fermentationsbehälter Belüftungsgase und/oder Reaktionsmedium zugeführt werden. Da ein derartiger Bioreaktor zur Erzeugung einer gleichmäßigen Fermentierung möglichst vollständig mit festen Stoffen befüllt sein muss, ist aufgrund der Größe des Bioreaktors die Menge der zu Fermentierenden Stoffe festgelegt.

Aus WO 00/29544 ist ein Bioreaktor zum Kultivieren von Mikroorganismen auf festen Stoffen bekannt. Der Bioreaktor besteht aus einer Vielzahl von Modulen, wobei die Module übereinanderstapelbar sind. Jedes Modul weist eine das Modul gegenüber dem benachbarten Modul abgrenzende Grundplatte mit Kanälen auf. Durch eine erste Art Kanäle ist eine Verbindung zwischen zwei benachbarten Modulen möglich. Eine zweite Art Kanäle, die quer zu der ersten Art Kanäle verläuft, ermöglicht keine Verbindung zwischen den Modulen. Ein derartiger Bioreaktor ist insbesondere aufgrund der zwei benachbarte Module verbindenden Kanäle zur Durchführung unterschiedlicher Fermentationen in den einzelnen Modulen nicht geeignet.

Aufgabe der Erfindung ist es, einen Bioreaktor zur Fermentation flüssiger und/oder fester Stoffe zu schaffen, mit denen auf einfache Weise parallel mehrere Fermentationen durchgeführt werden können.

Es wurde gefunden, dass mit speziellen Trennwänden und weiteren Modifikationen am Reaktorkörper die gestellte Aufgabe gelöst werden kann. Die Anmeldung betrifft somit
(1) einen Bioreaktor zur Fermentation flüssiger und/oder fester Stoffe, mit mindestens einer Trennwand (10) zur Unterteilung des Bioreaktors (14) in mindestens zwei Reaktionskammern (12), wobei jede Reaktionskammer (12) einen Auslass (16) aufweist und die Auslässe (16) der Reaktionskammern (12) unabhängig voneinander mit einer gemeinsamen Messkammer (20) verbunden sind und die Auslässe unabhängig voneinander öffen und schließbar sind; und
(2) ein Fermentationsverfahren mit wenigstens zwei parallelen Reaktionsansätzen, dadurch gekennzeichnet, dass es in einem Bioreaktor, wie in (1) beschrieben, erfolgt.

Der Bioreaktor gemäß Ausführungsform (1) der Erfindung weist mindestens eine Trennwand auf, durch die der Bioreaktor in mindestens zwei Reaktionskammern unterteilt werden kann. Jede Reaktionskammer weist einen gesonderten Auslass auf. Es ist somit möglich, unabhängig voneinander aus jeder einzelnen Reaktionskammer beispielsweise Proben zu entnehmen. Da derartige Proben beispielsweise hinsichtlich des PH-Werts und des Sauerstoffwertes in einer hierzu geeigneten Messkammer mit entsprechenden Messelektroden untersucht werden müssen, wäre es erforderlich, mit jedem Auslass einer Reaktionskammer auch eine Messkammer zu verbinden, um ein automatisches Zuführen der entnommenen Probe zu der Messkammer zu verwirklichen. Da derartige Messelektroden teuer sind, und insbesondere beim Vorsehen von einer Vielzahl von Reaktionskammern der apparative Aufwand sehr hoch ist, ist erfindungsgemäß eine einzige gemeinsame Messkammer vorgesehen. Hierbei ist jeder Auslass der Reaktionskammern mit der Messkammer verbunden. Dadurch ist es möglich, Proben aus den einzelnen Reaktionskammern zu entnehmen und automatisch der Messkammer zuzuführen. Um die Proben aus den einzelnen Reaktionskammern unabhängig voneinander untersuchen zu können, ist jeder Auslass vorzugsweise mit unabhängig voneinander öffen- und schließbaren Ventilen o.dgl. verbunden.

Vorzugsweise sind die Trennwände zur Unterteilung des Bioreaktors in mindestens zwei Reaktionskammern beweglich. Hierdurch ist es möglich, das Volumen der einzelnen Reaktionskammern zu variieren. Vorzugsweise können die Trennwände vollständig aus dem Bioreaktor herausgenommen und vorzugsweise in einem fest vorgegebenen Raster oder an beliebigen Stellen des Bioreaktors wieder eingesetzt werden. Es ist somit auf einfache Weise möglich, bei dem erfindungsgemäßen Bioreaktor Reaktionskammern mit unterschiedlichen Volumina vorzusehen.

Um ein Vermischen von aus unterschiedlichen Reaktionskammern entnommenen Proben zu vermeiden, ist die Mischkammer vorzugsweise mit einem Reinigungsflüssigkeit enthaltenden Reinigungsbehälter verbunden. Nach der Entnahme einer Probe aus einer Reaktionskammer kann diese beispielsweise durch einen Auslass aus der Messkammer abgeführt werden und anschließend die Messkammer mit Hilfe der Reinigungsflüssigkeit, vorzugsweise automatisch gereinigt werden. Hierdurch erfolgt ein Rekalibrieren der Messkammer, so dass nach dem Waschvorgang eine sich ggf. stark von der zuvor gemessenen Probe unterscheidende Probe aus einer anderen Reaktionskammer untersucht werden kann.

Vorzugsweise wird eine Reinigungsflüssigkeit verwendet, die zumindest in geringen Mengen auch den Reaktionskammern zugeführt werden kann, ohne den Fermentationsprozess in den einzelnen Reaktionskammern zu beeinträchtigen. Hierbei wäre eine geringfügige Beeinträchtigung des Fermentationsprozesses akzeptabel, insbesondere wenn während der Fermentation ein gutes Durchmischen der in der Reaktionskammer vorhandenen Stoffe sichergestellt ist. Durch Verwenden einer derartigen Reinigungsflüssigkeit ist es möglich, beim Reinigen der Mischkammer die der Reaktionskammer entnommene Probe wieder in die Reaktionskammer zurückzudrücken. Hierbei wird ggf. ein geringer Teil an Reinigungsflüssigkeit der Reaktionskammer zugeführt. Vorzugsweise ist zum Zurückführen der in der Messkammer enthaltenen Probe eine Druckerzeugungseinrichtung wie eine Pumpe vorgesehen, durch die die Reinigungsflüssigkeit derart unter Druck gesetzt werden kann, dass die einer Reaktionskammer entnommene Probe in die Reaktionskammer zurückgedrückt wird. Ein derartiges Vorgehen hat den Vorteil, dass gleichzeitig mit dem Reinigen der Messkammer auch eine zwischen einer Reaktionskammer und der Messkammer angeordnete Rohrleitung gereinigt wird. Dies hat wiederum den Vorteil, dass bei einer erneuten Probenentnahme aus derselben Reaktionskammer die entnommene Probe nicht aufgrund von in der Rohrleitung vorhandenen Resten der zuvor entnommenen Probe verfälscht wird. Ferner hat das Zurückdrücken der entnommenen Probe den Vorteil, dass der Auslass bzw. die Auslassöffnung der Reaktionskammer durch das Zurückdrücken der Probenflüssigkeit gereinigt wird.

Es ist ferner möglich, die entnommene Probe über eine externe Rohrleitung o.dgl. der Reaktionskammer wieder zuzuführen. Dies kann ggf. auch mit dem Reinigungsprozess der Messkammer verbunden werden.

Bei einer besonders bevorzugten Ausführungsform der Erfindung ist in jeder Reaktionskammer eine Düsenanordnung vorgesehen. Die Düsenanordnung weist vorzugsweise mehrere Leitungsrohre mit am Ende und/oder über die Länge verteilten Düsen auf. Mit Hilfe der Düsenanordnung kann in die einzelnen Reaktionskammern Gas zur Belüftung der Reaktionskammern oder Medium zur Beeinflussung des Fermentationsprozesses zugeführt werden. Die Düsenanordnung ist vorzugsweise mit einer druckdichten Platte verbunden, die gleichzeitig als Deckel des Bioreaktors dient. Besonders bevorzugt ist es, die Düsenanordnung derart auszugestalten, dass sie in die einzelnen Reaktionskammern ein- und ausfahrbar ist. Hierdurch ist ein Bestücken der Reaktionskammern erheblich vereinfacht. Vorzugsweise sind sämtliche Düsenanordnungen gemeinsam ein- und ausfahrbar. Beim Einfahren der Düsenanordnung in die einzelnen Reaktionskammern erfolgt somit vorzugsweise ein druckdichtes Verschließen der einzelnen Reaktionskammern durch die die Düsenanordnungen tragende druckdichte Platte. Das Verschieben der Düsenanordnung erfolgt vorzugsweise pneumatisch. Das herausfahrbare Ausgestalten der Düsenanordnungen hat den Vorteil, dass auf einfache Weise Leitungsrohre der Düsenanordnung ausgewechselt werden können. Dies ist beispielsweise bei Beschädigung einzelner Düsen erforderlich. Ferner können in Abhängigkeit des durchgeführten Fermentationsprozesses unterschiedliche Düsenanordnungen auf einfache Weise vorgesehen werden. Hierdurch kann beispielsweise auf einfache Weise die Anzahl der Düsen pro Leitungsrohr variiert werden.

Bei einer weiteren bevorzugten Ausführungsform weist der erfindungsgemäße Bioreaktor mehrere Reaktionskammern, insbesondere mehr als drei und besonders bevorzugt mehr als sechs Reaktionskammern auf. Die einzelnen Reaktionskammern sind durch Trennwände voneinander abgetrennt. Vorzugsweise sind die Trennwände auswechselbar ausgestaltet. Dies hat den Vorteil, dass die Größe der einzelnen Reaktionskammern auf einfache Weise variiert werden kann. Weist ein Bioreaktor beispielsweise zehn Kammern mit je 1,5 l Volumen auf, können durch Herausnehmen einzelner Trennwände Reaktionskammern mit beispielsweise 3 l, 4,5 l, 6 l usw. Volumen realisiert werden.

Die Trennwände und/oder der Bioreaktor weisen vorzugsweise doppellippige Dichtungen auf, so dass benachbarte Reaktionskammern vollständig voneinander abgegrenzt sind. Das Material der Dichtung ist hierbei inpermeabel für feste, flüssige und gasförmige Stoffe.

Bei einer bevorzugten Ausführungsform ist die Lage der Trennwände durch zwischen benachbarten Trennwänden angeordnete Abstandshalter definiert. Vorzugsweise sind im unteren Bereich des Bioreaktors untere Abstandshalter und im oberen Bereich des Bioreaktors obere Abstandshalter vorgesehen. Durch Vorsehen von Abstandshaltern unterschiedlicher Länge ist es ferner möglich, die Größe der einzelnen Reaktionskammern zu variieren. Es ist somit möglich, nicht nur Reaktionskammern auszubilden, die ein Vielfaches der kleinstmöglichen Reaktionskammer als Volumen enthalten, sondern auch eine beliebige Anzahl an Zwischenvolumina zu realisieren. Die Größe des Volumens ist hierbei nur von der Länge der Abstandshalter sowie der Lage der einzelnen Auslässe abhängig. Bei dieser Ausführungsform sind die Dichtungen vorzugsweise an der Trennwand selbst vorgesehen, so dass durch Einsetzen entsprechender Abstandshalter und einer Trennwand eine Reaktionskammer mit dem gewünschten Volumen ausgebildet ist.

Der untere Abstandshalter ist vorzugsweise derart angeordnet, dass er nicht nur als Abstandshalter zwischen zwei benachbarten Trennwänden bzw. zu einer Seitenwand des Bioreaktors, sondern auch als Verstopfungsschutz für den Auslass dient. Hierzu ist der Abstandshalter vorzugsweise derart ausgebildet, dass der Auslass innerhalb des unteren Abstandshalters angeordnet ist und der Abstandshalter eine Verbindungsöffnung, wie beispielsweise einen Schlitz aufweist, durch den eine zu entnehmende Probe im inneren Bereich des Abstandshalters eindringen und aus diesem über die Auslassöffnung abgelassen werden kann. Es ist somit verhindert, dass größere Stoffpartikel in die Messkammer gelangen und/oder den Auslass der Reaktionskammer verstopfen.

Vorzugsweise ist der erfindungsgemäße Bioreaktor zumindest teilweise doppelwandig ausgebildet, um ein Temperieren der zu Fermentierenden Stoffe zu ermöglichen. Hierbei kann der doppelwandige Bioreaktor einzelne Kammern aufweisen, so dass die einzelnen Reaktionskammern unterschiedlich temperiert werden können. Um Wärmeübergänge zwischen den einzelnen Reaktionskammern durch die Trennwände, die vorzugsweise aus Edelstahl sind, zu vermeiden oder zumindest zu verringern, handelt es sich bei dieser Ausführungsform vorzugsweise um wärmeisolierte Trennwände. Beispielsweise handelt es sich um doppelwandige Trennwände, wobei zwischen den Trennwänden vorzugsweise ein Vakuum besteht, um den Wärmeübergang zwischen zwei benachbarten Reaktionskammern zu verringern.

Gemäß Ausführungsform (2) der Erfindung ist der erfindungsgemäße Bioreaktor für Fermentationsverfahren mit wenigstens zwei parallelen Reaktionsansätzen geeignet. Es können hierbei flüssige und/oder feste Stoffe fermentiert werden. Bei dem erfindungsgemäßen Verfahren können zum einen die Volumina der Reaktionsansätze gleich groß sein, sodass bei Variation der Verfahrensparameter wie Temperatur, pH, Nährstoffgehalt, Gaskonzentration (z. B. O₂), mögliche Induktoren, Proteinkonzentrationen, Zellzahl usw., deren Einfluss auf den Ablauf der Fermentation getestet werden kann, d. h. in der Messkammer direkt beobachtet werden kann. Anderseits ist auch eine asymmetrische Teilung des Reaktionsvolumens möglich, d. h. unterschiedlich große Reaktionsvolumina, wobei das "Upscaling" einer Fermentationsreaktion simuliert werden kann. Das Fermentationsverfahren gemäß Ausführungsform (2) der Erfindung ist somit zum parallelen Optimieren der Reaktionsbedingungen einer Fermentationsreaktion geeignet. Die parallelen Fermentationsreaktionen können einerseits durch *in-situ-*Messung von einem oder mehreren für die entsprechende Reaktion indikative Verfahrensparameter(n) (wie die oben genannten) in der Messkammer des Reaktors verfolgt werden. Weitere Informationen sind durch konventionelle Analyse der Fermentationslösung während oder nach Beendigung der Fermentationsreaktion zugänglich. Die *in-situ*-Messung von Verfahrensparametern in einer Messkammer erfordert dabei auf die jeweilige Fermentationsreaktion angepasste Wasch- und Kalibrierschritte der Messkammer, wie vorstehend erwähnt.

Nachfolgend wird die Erfindung anhand einer bevorzugten Ausführungsform unter Bezugnahme auf die anliegenden Zeichnungen näher erläutert.

Es zeigen:
- Fig. 1: eine schematische Seitenansicht einer bevorzugten Ausführungsform eines erfindungsgemäßen Bioreaktors,
- Fig. 2: eine schematische Seitenansicht des Bioreaktors in Richtung der Pfeile II in Fig. 1,
- Fig. 3: eine schematische Schnittansicht entlang der Linie III-III in Fig. 1,
- Fig. 4: eine schematische Schnittansicht entlang der Linie IV-IV in Fig. 2, und
- Fig. 5: eine schematische Teilschnittansicht entlang der Linie V-V in Fig. 3.

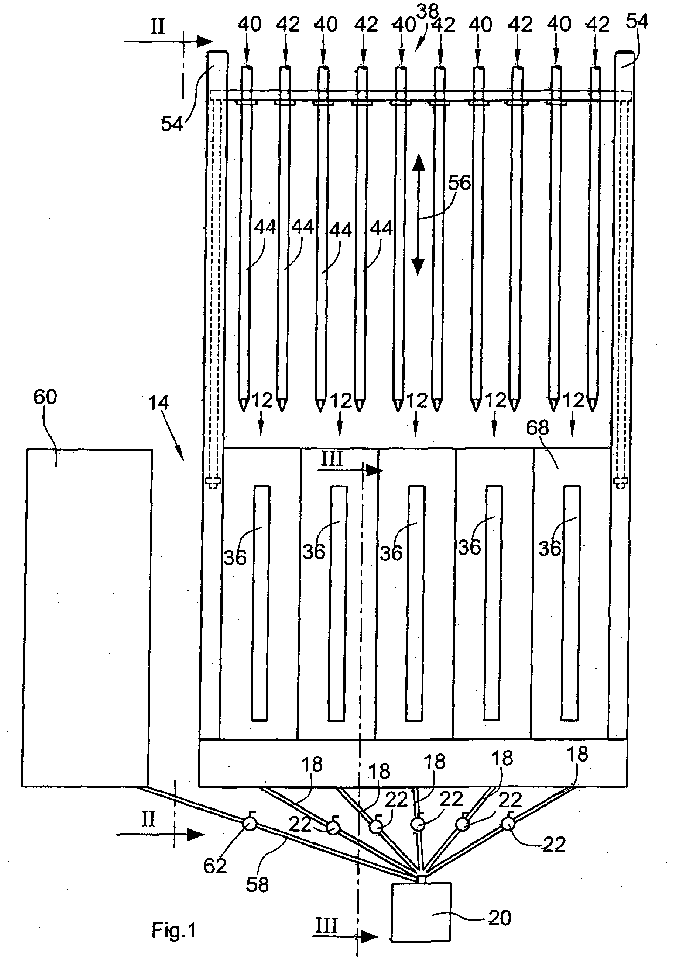

Der erfindungsgemäße Bioreaktor ist durch Trennwände 10 in mehrere Reaktionskammern 12 (Fig. 1) unterteilt. In dem dargestellten Ausführungsbeispiel handelt es sich um fünf nebeneinander angeordnete Reaktionskammern mit gleichem Volumen. Durch Herausnehmen einer oder mehrerer Trennwände 10 aus dem Bioreaktor 14 lässt sich die Größe der einzelnen Volumina der Reaktionskammern 12 variieren. Bei einem Volumen von beispielsweise 1,5 l je dargestellte Reaktionskammer 12 (Fig. 1) lassen sich Volumina von 1,5 l, 3 l, 4,5 l, 6 l und 7,5 l herstellen.

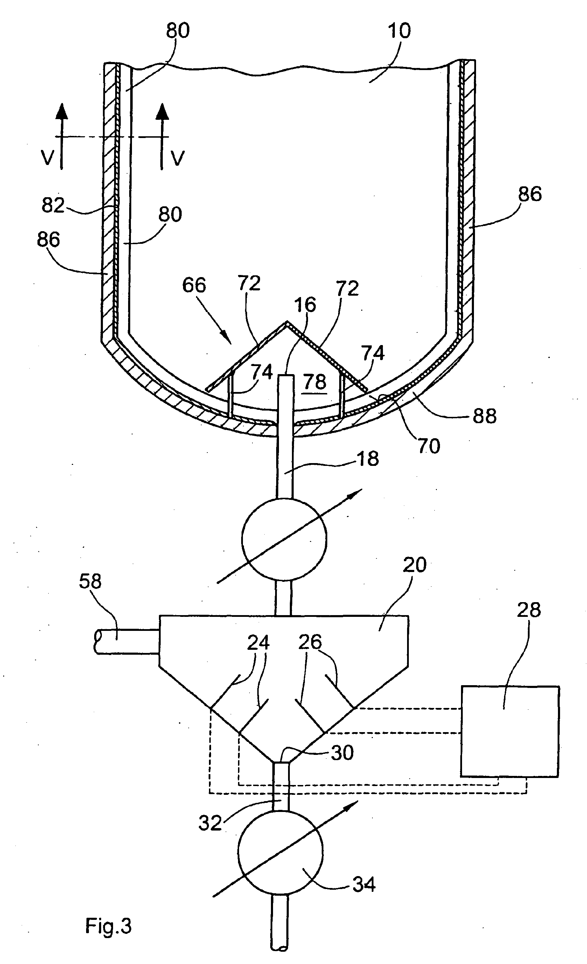

Jede einzelne Reaktionskammer 12 ist über eine Auslassöffnung 16 (Fig. 3) und ein die Auslassöffnung 16 aufweisendes Rohr 18 mit einer Messkammer 20 verbunden. In jedem Rohr 18 ist ein öffen- und schließbares Ventil 22 angeordnet. Durch Öffnen eines der Ventile 22 kann eine Probe aus einer Reaktionskammer 12 entnommen und der Messkammer 20 zugeführt werden.

In der Messkammer 20 sind im dargestellten Ausführungsbeispiel zwei Elektrodenpaare 24,26 vorgesehen, die beispielsweise zur Messung des PH-Wertes oder des Sauerstoffgehaltes dienen. Die beiden Elektrodenpaare 24,26 sind elektrisch mit einer Auswerteeinheit 28 zur Auswertung der Messergebnisse verbunden. Wie in Fig. 3 dargestellt, kann die Messkammer beispielsweise kegelförmig ausgebildet sein und an ihrem tiefsten Punkt eine Auslässöffnung 30 zum Ablassen einer in der Messkammer 20 gemessenen Probe aufweisen. Die Auslassöffnung 30 ist mit einem Rohr 32 und einem Absperrventil 34 verbunden. Die Möglichkeit die Probe durch eine Auslassöffnung 30 abzulassen, kann auch entfallen, wobei die in der Messkammer 20 enthaltende Probe sodann wie später beschrieben in die entsprechende Reaktionskammer 12 zurückgedrückt wird.

Beispielsweise zum optischen Untersuchen der in den Reaktionskammern 12 stattfindenden Reaktionen mit Hilfe eines optischen Sensors weist jede Reaktionskammer 12 ein Schauglas 36 (Fig. 1) auf.

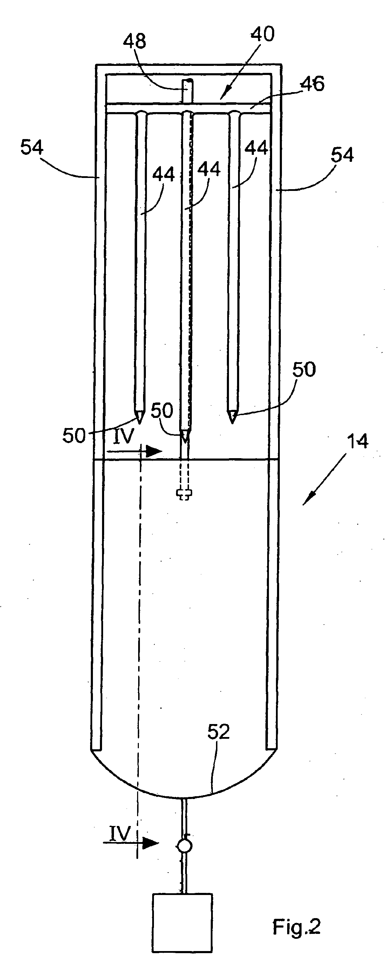

Die einzelnen Reaktionskammern 12 können in Fig. 1 von oben mit zu fermentierenden flüssigen oder festen Stoffen befüllt werden. Die Zugabe von flüssigem oder gasförmigem Reaktionsmedium erfolgt durch eine Düsenanordnung 38. Die Düsenanordnung 38 weist in dem dargestellten Ausführungsbeispiel je Reaktionskammer 12 zwei Düseneinrichtungen 40,42 mit jeweils drei Leitungsrohren 44 auf (Fig. 2). Jeweils drei Leitungsrohre 44 einer Düseneinrichtung 40,42 sind über eine gemeinsame Querleitung 46 miteinander verbunden. Über einen rohrförmigen Ansatz 48 kann jede einzelne Düseneinrichtung 40,42 mit einem Anschluss zum Zuführen von Medium und/oder Luft verbunden werden. Am Ende jedes Düsenrohrs 44 sind Düsen 50 vorgesehen, durch die das Medium innerhalb der Reaktionskammer 12 verteilt wird. Um die Düsenanordnung 38 möglichst tief in die Reaktionskammern 12 einführen zu können, weisen die Leitungsrohre 44 unterschiedliche Längen auf, die entsprechend an die Form eines Bodens 52 des Bioreaktors 14 angepasst sind.

Die gesamte Düsenanordnung 38 ist in vier Führungen 54, die an den Ecken des Bioreaktors 14 angeordnet sind, in Richtung eines Pfeils 56 verschiebbar gehalten. Das Verschieben erfolgt beispielsweise über eine geeignete pneumatische oder hydraulische Einrichtung. Die gesamte Düsenanordnung ist somit in den Bioreaktor 14, d.h. in die einzelnen Reaktionskammern 12 ein- und ausfahrbar. Dies erleichtert beispielsweise das Bestücken der einzelnen Reaktionskammern 12 mit zu fermentierenden Stoffen. Ferner kann die gesamte Düsenanordnung 38 z. B. zum Reinigen des Bioreaktors entfernt werden.

Zur Entnahme von Proben aus einer der Reaktionskammern 12 wird eines der Ventile 22 geöffnet, so dass die zu entnehmende Probe durch die Rohrleitung 18 in die Messkammer 20 fließt. Ebenso können den einzelnen Kammern 12 über einen weiteren Anschluss an die Rohrleitung 18 Medien oder Druckgase zugegeben werden. Hierdurch ist es möglich, den Bioreaktor beispielsweise als Air Lift Reaktor zu betreiben. Das Düsensystem 38 ist beim Betreiben des Bioreaktors als Air Lift Reaktor optional. Nach dem Entleeren der Messkammer 20, beispielsweise über die Auslassöffnung 30 erfolgt ein Reinigen der Messkammer 20. Hierzu ist die Messkammer 20 über eine Rohrleitung 58 mit einem Reinigungsbehälter 60, in dem Reinigungsflüssigkeit enthalten ist, verbunden. Zum Reinigen wird ein Ventil 62 geöffnet. Ggf. erfolgt das Zuführen der Reinigungsflüssigkeit durch die Rohrleitung 58 in die Messkammer 20 mit Druck, um ein gründliches Reinigen der Messkammer 20 sicherzustellen. Durch das Reinigen mit einer geeigneten Reinigungsflüssigkeit erfolgt eine Rekalibrierung der Messkammer 20.

Anstatt des Ablassens der in der Messkammer 20 enthaltenen Probe durch eine Auslassöffnung 30 kann ein Zurückdrücken der in der Messkammer 20 enthaltenen Probe mit Hilfe der durch die Rohrleitung 58 zugeführte Reinigungsflüssigkeit erfolgen. Hierbei wird die Probe durch die Rohrleitung 18, durch die sie aus der entsprechenden Reaktionskammer 12 entnommen wurde, wieder in dieselbe Reaktionskammer 12 zurückgedrückt. Ferner ist es möglich, durch einen beispielsweise zusätzlichen Anschluss an der Messkammer 20 über diese eine beliebige andere Flüssigkeit einer bestimmten Reaktionskammer 12 zuzuführen.

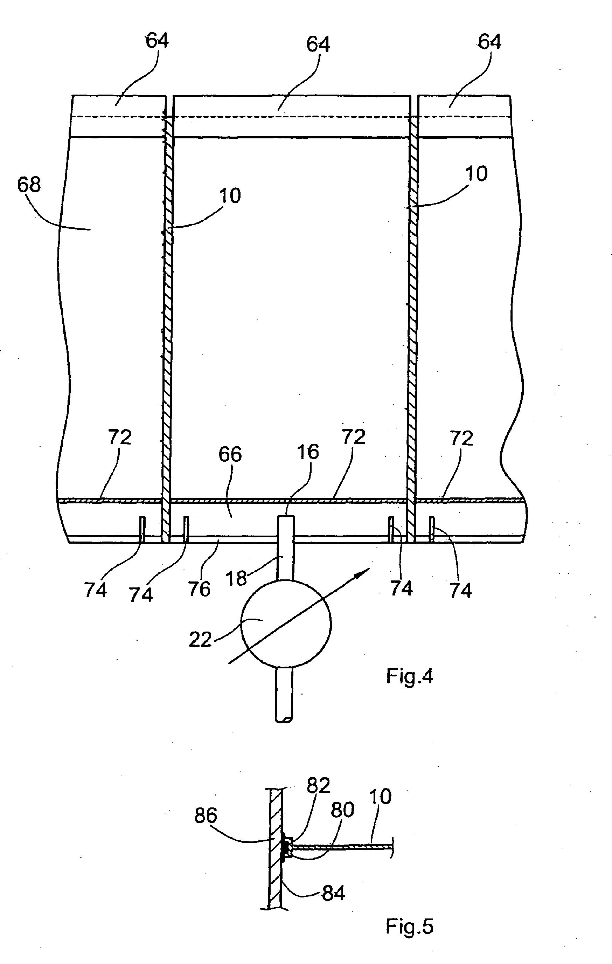

Die einzelnen Trennwände 10 sind jeweils durch einen oberen Abstandshalter 64 und einen unteren Abstandshalter 66 (Fig. 4) auf Abstand gehalten. Der obere Abstandshalter 64 ist beispielsweise im Querschnitt U-förmig und wird von oben über eine Seitenwand 68 des Bioreaktors 14 gesteckt. Der untere Abstandshalter 66 weist dieselbe Breite wie der obere Abstandshalter 64 auf und wird auf einen Boden 70 des Bioreaktors 14 gelegt.

Der untere Abstandshalter 66 besteht im dargestellten Ausführungsbeispiel aus zwei in einem Winkel zueinander angeordneten ebenen Teilen 72. Die beiden ebenen Teile 72 sind beispielsweise durch ein abgekantetes Blech aus Edelstahl o.dgl. gebildet und bilden somit eine Art Dach. An der in Richtung des Bodens 70 weisenden Unterseite der beiden ebenen Teile 72 sind mindestens vier stabförmige Abstandshalter 74 vorgesehen. Die Abstandshalter 74 erstrecken sich nicht über die gesamte Breite der Reaktionskammer 12, so dass zwischen den Abstandshaltern 74 ein Schlitz 76 ausgebildet ist. Durch diesen Schlitz 76 kann durch die Fermentation entstehende Reaktionsflüssigkeit in eine innerhalb des unteren Abstandshalters 66 angeordnete Sammelkammer 78 gelangen. Mit der Sammelkammer 78 ist der Auslass 16 verbunden bzw. das Ende des Rohrs 18 ist innerhalb der Sammelkammer 78 angeordnet.

Um die Trennwände 10 benachbarter Reaktionskammern 12 gegeneinander abzudichten, weist jede Trennwand 10 am gesamten Umfang einen U-förmigen Ansatz 80 (Fig. 5) auf, in dem eine Dichtung 82 angeordnet ist. Die Dichtung 82 liegt an einer Innenseite 84 der beiden Seitenwände 86 sowie der Bodenwand 88 an. Ferner können die einzelnen Reaktionskammern 12 durch einen Deckel dicht verschlossen werden. An diesem liegt die Dichtung 82 ebenfalls dicht an.

Die Verwendung des erfindungsgemäßen Bioreaktors zur Durchführung meherer paralleler Reaktionsansätze ist weiterhin in dem nachfolgenden Ausführungsbeispiel beschrieben.

### Beispiel

Hefefermentation: In einem erfindungsgemäßen Bioreaktor mit 10 Kammern mit jeweils einem Volumen von 1,5 l und einer Messkammer wurde die Bäckerhefe Saccharomyces cerevisiae bei zehn verschiedenen pH-Werten (von 2, 2,7, 3,4, 4,1, 4,8, 5,5, 6,2, 6,9, 7,6, 8,3) für 24 Stunden bei 30 °C in einem Unversal-Hefemedium kultiviert und das Wachstum anhand der Zellzahl bestimmt. Der pH-Wert jeder Kammer wurde im Abstand von 30 Minuten in der Messkammer gemessen und bei Abweichungen zum Sollwert gegen titriert. Die Durchmischung und Belüftung erfolgte in allen Kammern gleich über das Düsensystem 38 und zusätzlich über eine zweite Zuleitung durch Rohrleitung 18. Bestes Wachstum, bis zu 10⁸ Zellen im ml, wurde bei einem pH von 4,8 erzielt.

## Patentansprüche

1. Bioreaktor zur Fermentation flüssiger und/oder fester Stoffe, mit mindestens einer Trennwand (10) zur Unterteilung des Bioreaktors (14) in mindestens zwei Reaktionskammern (12), wobei jede Reaktionskammer (12) einen Auslass (16) aufweist, die Auslässe (16) der Reaktionskammern (12) unabhängig voneinander mit einer gemeinsamen Messkammer (20) verbunden sind und die Auslässe unabhängig voneinander öffen- und schließbar sind.

2. Bioreaktor nach Anspruch 1, **dadurch gekennzeichnet, dass** die Lage der Trennwände (10) zur Volumenveränderung der Reaktionskammern (12) veränderbar ist.

3. Bioreaktor nach Anspruch 1 oder 2, **dadurch gekennzeichnet, dass** die Messkammer (20) mit einem eine Reinigungsflüssigkeit enthaltenden Reinigungsbehälter (60) verbunden ist.

4. Bioreaktor nach einem der Ansprüche 1-3, **dadurch gekennzeichnet, dass** die Messkammer (20) mit einer Druckerzeugungseinrichtung zum Zurückführen entnommener Proben in die Reaktionskammer (12) verbunden ist.

5. Bioreaktor nach Anspruch 4, **dadurch gekennzeichnet, dass** die Druckerzeugungseinrichtung über eine vom Reinigungsbehälter (60) zur Messkammer (20) führenden Rohrleitung (58) mit der Messkammer (20) verbunden ist, wobei das Zurückdrücken der Probe durch Reinigungsflüssigkeit erfolgt.

6. Bioreaktor nach einem der Ansprüche 1 - 5, **gekennzeichnet durch** eine Düsenanordnung (38) zum Zuführen von flüssigem oder gasförmigem Medium zu den einzelnen Reaktionskammern (12).

7. Bioreaktor nach Anspruch 6, **dadurch gekennzeichnet, dass** die Düsenanordnung (38) je Reaktionskammer (12) mindestens eine Düseneinrichtung (40,42) aufweist.

8. Bioreaktor nach Anspruch 7, **dadurch gekennzeichnet, dass** je Reaktionskammer mindestens eine Düseneinrichtung ist (40,42) zur getrennten Zuführung von Medien vorgesehen ist.

9. Bioreaktor nach einem der Ansprüche 1-8, **dadurch gekennzeichnet, dass** die Düseneinrichtungen (40,42) in die Reaktionskammern (12) insbesondere gemeinsam ein- und ausfahrbar sind.

10. Bioreaktor nach einem der Ansprüche 1 - 9, **dadurch gekennzeichnet, dass** die Trennwand (10) und/oder der Bioreaktor (14) doppellippige Dichtungen (80) zur Abdichtung der einzelnen Reaktionskammern (12) aufweist.

11. Bioreaktor nach einem der Ansprüche 1 - 10, **dadurch gekennzeichnet, dass** die Lage der Trennwand (10) durch zwischen benachbarten Trennwänden (10) angeordneten Abstandshaltern (64,66) definiert ist.

12. Bioreaktor nach Anspruch 11, **dadurch gekennzeichnet, dass** ein unterer Abstandshalter (66) zusätzlich als Verstopfungsschutz für den Auslass (16) dient.

13. Bioreaktor nach Anspruch 12, **dadurch gekennzeichnet, dass** der Auslass (16) innerhalb des unteren Abstandshalters (66) angeordnet ist und der Abstandshalter eine Verbindungsöffnung (76) zu der Reaktionskammer (12) aufweist.

14. Bioreaktor nach Anspruch 11 oder 13, **dadurch gekennzeichnet, dass** innerhalb des Abstandshalters (66) ein Probensammelraum (78) für flüssige Proben vorgesehen ist.

15. Bioreaktor nach einem der Ansprüche 1 - 14, **dadurch gekennzeichnet, dass** der Bioreaktor (14) zur Temperierung mit Fluiden zumindest teilweise doppelwandig ist.

16. Fermentationsverfahren mit wenigstens zwei parallelen Reaktionsansätzen, **dadurch gekennzeichnet, dass** es in einem Bioreaktor gemäß einem oder mehreren der Ansprüche 1-15 erfolgt.

17. Fermentationsverfahren gemäß Anspruch 16, wobei die Reaktionsvolumina der parallelen Reaktionsansätze gleich groß oder unterschiedlich groß sind.

18. Fermentationsverfahren gemäß Anspruch 16 oder 17, wobei sich die Reaktionsansätze hinsichtlich eines Reaktionsparameters, insbesondere Temperatur, pH, Nährstoffgehalt, Gaskonzentration, Anwesenheit möglicher Induktoren, Proteinkonzentration und/oder Zellzahl unterscheiden.

19. Fermentationsverfahren gemäß einem oder mehreren der Ansprüche 16 - 18, das zum parallelen Optimieren der Reaktionsbedingungen einer Fermentationsreaktion geeignet ist.

20. Fermentationsverfahren nach Anspruch 19, welches zum Upscalen der Fermentationsreaktion geeignet ist.

## Claims

1. A bioreactor for the fermentation of liquid and/or solid matter, comprising at least one partition wall (10) for partitioning the bioreactor (14) into at least two reaction chambers (12), each reaction chamber (12) having an outlet (16), the outlets (16) of the reaction chambers (12) being independently connected to a common measuring chamber (20), and the outlets being adapted to be opened and closed independently.

2. The bioreactor of claim 1, **characterized in that** the position of the partition walls (10) is variable to change the volume of the reaction chambers (12).

3. The bioreactor of claim 1 or 2, **characterized in that** the measuring chamber (20) is connected to a cleaning vessel (60) containing a cleaning liquid.

4. The bioreactor of one of claims 1-3, **characterized in that** the measuring chamber (20) is connected to a pressure generating means to return samples taken into the reaction chamber (12).

5. The bioreactor of claim 4, **characterized in that** the pressure generating means is connected to the measuring chamber (20) via a pipeline (58) leading from the cleaning, vessel (60) to the measuring chamber (20), the sample being pushed back by the cleaning liquid.

6. The bioreactor of one of claims 1-5, **characterized by** an array of nozzles (38) for feeding liquid or gaseous media to the individual reaction chambers (12).

7. The bioreactor of claim 6, **characterized in that** the array of nozzles (38) comprises at least one nozzle means (40, 42) per reaction chamber (12).

8. The bioreactor of claim 7, **characterized in that** at least one nozzle means (40, 42) is provided per reaction chamber for the separate supply of media.

9. The bioreactor of one of claims 1-8, **characterized in that** the nozzle means (40, 42) are adapted to be moved into and out of the reaction chambers (12), in particular together.

10. The bioreactor of one of claims 1-9, **characterized in that** the partition wall (10) and/or the bioreactor (14) comprise double-lipped seals (80) for sealing the individual reaction chambers (12).

11. The bioreactor of one of claims 1-10, **characterized in that** the position of the partition wall (10) is defined by spacers (64, 66) arranged between adjacent partition walls (10).

12. The bioreactor of claim 11, **characterized in that** a lower spacer (66) additionally serves as a plugging guard for the outlet (16).

13. The bioreactor of claim 12, **characterized in that** the outlet (16) is arranged within the lower spacer (66) and the spacer has a communication opening (76) to the reaction chamber (12).

14. The bioreactor of claim 11 or 13, **characterized in that** a sample collecting space (78) for liquid samples is provided within the spacer (66).

15. The bioreactor of one of claims 1-14, **characterized in that** the bioreactor (14) is at least partly double-walled for temperature regulation with fluids.

16. A fermentation method comprising at least two parallel reaction batches, **characterized in that** it is performed in a bioreactor of one or a plurality of claims 1-15.

17. The fermentation method of claim 16, wherein the reaction volumes of the parallel reaction batches are of equal or different sizes.

18. The fermentation method of claim 16 or 17, wherein the reaction batches differ in one reaction parameter, in particular the temperature, pH-value, nutrient content, gas concentration, presence of possible inductors, protein concentration and/or cell number.

19. The fermentation method of one or a plurality of claims 16-18, suited for a parallel optimization of the reaction conditions of a fermentation reaction.

20. The fermentation method of claim 19, suited for upscaling the fermentation reaction.

## Revendications

1. Bioréacteur pour la fermentation de substances liquides et/ou solides, comportant au moins une cloison (10) pour la subdivision du bioréacteur (14) en au moins deux chambres de réaction (12), chaque chambre de réaction (12) présentant une sortie (16), les sorties (16) des chambres de réaction (12) étant reliées, indépendamment l'une de l'autre, à une chambre de mesure commune (20), et les sorties pouvant être ouvertes et fermées indépendamment l'une de l'autre.

2. Bioréacteur selon la revendication 1, **caractérisé en ce que** la position des cloisons (10) est modifiable en vue de la modification du volume des chambres de réaction (12).

3. Bioréacteur selon la revendication 1 ou 2, **caractérisé en ce que** la chambre de mesure (20) est reliée à un réservoir de nettoyage (60) contenant un liquide de nettoyage.

4. Bioréacteur selon l'une des revendications 1 à 3, **caractérisé en ce que** la chambre de mesure (20) est reliée à un dispositif générateur de pression pour ramener dans la chambre de réaction (12), des échantillons prélevés.

5. Bioréacteur selon la revendication 4, **caractérisé en ce que** le dispositif générateur de pression est relié à la chambre de mesure (20) par l'intermédiaire d'un conduit (58) menant du réservoir de nettoyage (60) à la chambre de mesure (20), le refoulement de l'échantillon étant effectué au moyen de liquide de nettoyage.

6. Bioréacteur selon l'une des revendications 1 à 5, **caractérisé par** un ensemble d'injection (38) pour l'apport d'un agent liquide ou gazeux aux différentes chambres de réaction (12).

7. Bioréacteur selon la revendication 6, **caractérisé en ce que** l'ensemble d'injection (38) comprend au moins un injecteur (40, 42) par chambre de réaction (12).

8. Bioréacteur selon la revendication 7, **caractérisé en ce que** pour chaque chambre de réaction, il est prévu au moins un injecteur (40, 42) pour l'apport séparé d'agents.

9. Bioréacteur selon l'une des revendications 1 à 8, **caractérisé en ce que** les injecteurs (40, 42) peuvent être rentrés dans les chambres de réaction (12) et en être sortis, en particulier conjointement.

10. Bioréacteur selon l'une des revendications 1 à 9, **caractérisé en ce que** la cloison (10) et/ou le bioréacteur (14) présente des éléments d'étanchéité à double lèvre (80) pour l'étanchéification des différentes chambres de réaction (12).

11. Bioréacteur selon l'une des revendications 1 à 10, **caractérisé en ce que** la position de la cloison (10) est définie par des entretoises (64, 66) disposées entre des cloisons voisines (10).

12. Bioréacteur selon la revendication 11, **caractérisé en ce qu'**une entretoise inférieure (66) sert, en plus, de protection anti-colmatage pour la sortie (16).

13. Bioréacteur selon la revendication 12, **caractérisé en ce que** la sortie (16) est située à l'intérieur de l'entretoise inférieure (66) et l'entretoise présente une ouverture de liaison (76) à la chambre de réaction (12).

14. Bioréacteur selon la revendication 11 ou 13, **caractérisé en ce qu'**à l'intérieur de l'entretoise (66) est prévu un espace collecteur d'échantillons (78) pour des échantillons liquides.

15. Bioréacteur selon l'une des revendications 1 à 14, **caractérisé en ce que** le bioréacteur (14) est, au moins partiellement, à double paroi, en vue d'un équilibrage de température avec des fluides.

16. Procédé de fermentation à l'aide d'au moins deux préparations réactionnelles parallèles, **caractérisé en ce qu'**il se déroule dans un bioréacteur conforme à une ou plusieurs des revendications 1 à 15.

17. Procédé de fermentation conforme à la revendication 16, dans lequel les volumes de réaction des préparations réactionnelles parallèles sont de même grandeur ou de grandeurs différentes.

18. Procédé de fermentation conforme à la revendication 16 ou 17, dans lequel les préparations réactionnelles se différencient par rapport à un paramètre de réaction, en particulier la température, le pH, la teneur en substances nutritives, la concentration de gaz, la présence d'inducteurs possibles, la concentration de protéines et/ou le nombre de cellules.

19. Procédé de fermentation conforme à une ou plusieurs des revendications 16 à 18, qui est approprié pour l'optimisation en parallèle des conditions réactionnelles d'une réaction de fermentation.

20. Procédé de fermentation selon la revendication 19, qui est approprié pour l'augmentation d'échelle de la réaction de fermentation.
